# NOUVEAU FASCICULE DE BREVET EUROPEEN

(11) **EP 2 635 238 B2**
(45) Date de publication et mention de la décision concernant l'opposition: **03.07.2019**
(45) Mention de la délivrance du brevet: 27.04.2016
(21) Numéro de dépôt: 11785739.1
(22) Date de dépôt: 21.10.2011
(51) Int. Cl.: A61F 2/40, A61F 2/46, A61B 17/17, A61B 34/00

(54) **GABARIT DE POSE D'UNE PROTHESE D'EPAULE SUR UNE GLENE**
EINSPANNVORRICHTUNG ZUR POSITIONIERUNG EINER SCHULTERPROTHESE AUF EINEM SCHAFT
JIG FOR PLACING A SHOULDER PROSTHESIS ONTO A SOCKET

(30) Priorité: 05.11.2010 FR 1059147
(43) Date de publication de la demande: 11.09.2013
(73) Titulaire: Aston Medical, 42000 Saint Etienne (FR)
(72) Inventeur: TROUILLOUD, Pierre, 21000 Dijon (FR); GONZALVEZ, Martin, 21000 Dijon (FR); ALEPEE, Christophe, 69003 Lyon (FR)
(74) Mandataire: Cabinet Laurent & Charras
(86) Numéro de dépôt international: PCT/FR2011/052461
(87) Numéro de publication internationale: WO 2012/059661

(56) Documents cités:
- EP-A1- 1 813 215
- WO-A1-2005/051209
- WO-A1-2011/060536
- WO-A1-2011/110374
- FR-A1- 2 898 267
- US-A1- 2004 193 175
- US-A1- 2006 074 353
- US-A1- 2008 243 127
- US-A1- 2010 082 035

## Description

L'invention se rattache au secteur technique des instruments pour la pose d'implants orthopédiques.

Plus particulièrement, l'invention concerne un guide ou gabarit sur mesure pour la pose d'une prothèse d'épaule, notamment du type inversé, en ce que la tête d'articulation en tant que telle est fixée au niveau de la glène (cavité glénoïde) pour coopérer avec une cupule de forme complémentaire que présente l'élément huméral de la prothèse. D'autres types de prothèses peuvent être envisagées telles que les prothèses anatomiques, les prothèses de resurfaçage....

Ce type de prothèse ressort par exemple de l'enseignement du brevet FR 2618065. La tête d'articulation peut être fixée dans la cavité glénoïde soit directement, soit par l'intermédiaire d'une embase support. Or, il est apparu que la fixation et le positionnement de cette embase support recevant la tête d'articulation ou directement la cupule s'avèrent particulièrement importants pour la réussite du fonctionnement biomécanique de la prothèse d'épaule et la durée de vie de l'implant quel que soit son type. Généralement, le support ou autre est fixé dans la cavité glénoïde au moyen d'une instrumentation chirurgicale standard.

Le document US 2006 074 353 décrit un gabarit d'une prothèse d'épaule sur une glène avec des parties d'appui aptes à coopérer avec les rebords de la glène.

Une étape importante de la procédure de pose consiste à orienter le perçage d'un trou dans la cavité glénoïde afin de déterminer l'axe de fraisage qui conditionne le positionnement de la prothèse.

Généralement, un praticien utilise un guide de perçage mais dont la conception ne permet pas de reproduire précisément une planification préliminaire. En effet, selon l'état de la technique, préalablement à la mise en place de la prothèse, le chirurgien planifie la chirurgie à partir d'une image bidimensionnelle de l'articulation au moyen par exemple d'une radiographie, d'un scanner ou d'une IRM. Des études ont démontré qu'il est particulièrement important de positionner le support glénoïdien de la prothèse sur la cavité glénoïde par rapport à des points de repères anatomiques précis, à partir desquels des mesures pourront être effectuées. Or, les résultats obtenus dans le cas d'une planification à partir d'une imagerie bidimensionnelle demeurent imprécis et ne permettent pas d'atteindre les objectifs de la planification. Par exemple, l'orientation des coupes 2D à une incidence directe sur la mesure effectuée.

L'invention s'est fixée pour but de remédier à ces inconvénients d'une manière simple, sûre, efficace et rationnelle en planifiant en trois dimensions la pose de la prothèse à partir d'images provenant de scanners ou d'IRM. Dans ce but, les images 2D sont traitées aux moyens de logiciels spécifiques permettant de segmenter les zones osseuses d'intérêt pour la pose, en ayant pour objectif de reconstruire une structure tridimensionnelle. Sur cette structure sont définis des repères permettant de définir un système de coordonnées spécifique à l'omoplate. Ce modèle tridimensionnel permet de simuler, planifier et mesurer précisément l'orientation de la prothèse en fonction du système de coordonnées précité. Cette planification se traduit par la définition mathématique d'un axe de perçage défini par un vecteur et une origine. Autour de cet axe est conçu un guide ou gabarit de pose sur mesure pour restituer physiquement cet axe en per-opératoire pour permettre la pose optimale de l'implant.

Pour atteindre ces objectifs, le guide de pose selon l'invention, d'une prothèse d'épaule sur une glène, est conforme aux caractéristiques de la revendication 1. Il ressort de ces caractéristiques que les agencements de positionnement et de fixation temporaire ne sont pas orientés par rapport au système de coordonnées, mais uniquement définis par les formes des zones de contact sur des rebords postérieur et antérieur de la glène. Le manche (guide), quant à lui, est orienté en fonction de la planification dans le système de coordonnées de l'omoplate tel que défini.

Le guide ainsi construit s'adapte de manière unique sur la structure anatomique osseuse du patient.

Le gabarit de pose selon l'invention permet donc de de matérialiser le positionnement non réel, de l'axe de perçage planifié en référence aux zones anatomiques du patient.

Pour résoudre le problème posé d'assurer le positionnement du gabarit par rapport aux repères déterminés dans le cadre de la planification préparatoire tridimensionnelle, les agencements de positionnement et de fixation temporaire sont opposés et profilés pour assurer un effet de clipage sur les rebords de la glène.

Dans une forme de réalisation du gabarit de pose, pour résoudre le problème posé d'assurer le perçage de la glène à partir du gabarit de pose, la zone de liaison constitue un anneau de forme générale sensiblement circulaire. Le manche de préhension est relié à la zone de liaison par une portée tronconique. La portée tronconique et la base du manche présentent deux ouvertures opposées situées de part et d'autre des zones de contact constituées par les pattes.

Ces ouvertures peuvent, notamment, permettre un contrôle visuel du positionnement du gabarit, et le contrôle visuel du point de perçage. Elles permettent, également, l'obtention d'une élasticité entre les rebords postérieur et antérieur, facilitant la mise en place du gabarit sur la structure osseuse.

On observe également que le manche, qui intègre l'axe de perçage préalablement planifié, assure un positionnement stable de l'outil de perçage afin de réaliser ledit perçage selon la planification préalablement établie.

Il ressort des caractéristiques de l'invention, que le guide ou gabarit de pose s'adapte de manière précise sur la structure anatomique du patient constituant par conséquent un gabarit de pose sur mesure en ce sens que pour chaque patient, on réalise un nouveau gabarit.

L'invention est exposée ci-après plus en détails à l'aide des figures des dessins annexés dans lesquels :

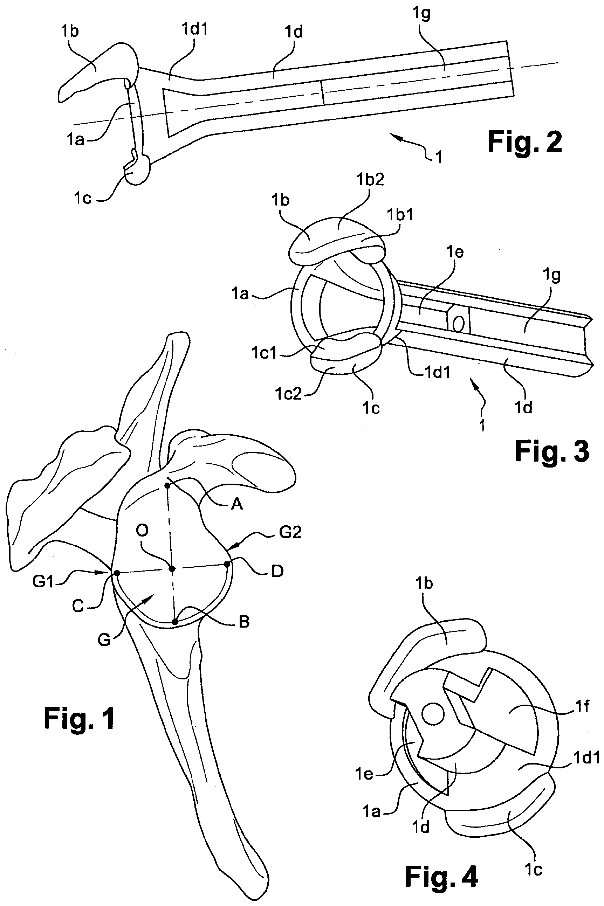

- la figure 1 montre une omoplate avec notamment les repères anatomiques au niveau de la cavité glénoïde.
- la figure 2 est une vue de face du gabarit de pose selon l'invention.
- la figure 3 est une vue en perspective du gabarit considéré par le dessous c'est-à-dire à partir des zones d'appuis.
- la figure 4 est une vue en perspective du gabarit considéré à partir du dessus, c'est-à-dire à partir du guide de perçage.
- la figure 5 montre la mise en place du gabarit de pose par rapport à la glène.

Comme indiqué, le gabarit de pose selon l'invention est réalisé à partir d'une planification peropératoire conçue sur une reconstruction tridimensionnelle faite à partir d'images provenant d'un scanner, d'IRM..., laquelle planification est résumée par la définition unique d'un axe de perçage, défini par un vecteur et une origine qui donnent l'orientation du gabarit de pose sur mesure, et à partir duquel sera positionné sur le gabarit de pose sur mesure.

Il est donc particulièrement important de définir le centre (O) de la glène (G) qui est défini à partir de 4 points qui sont respectivement constitués par le rebord supérieur de la glène (A), au pied de la coracoïde le rebord inférieur de la glène (B), le rebord postérieur (C) en regard de l'épine de l'acromion de la glène , et le rebord antérieur (D) de la glène . Ainsi, le croisement des deux droites (A-B) et (C-D) défini le centre (O) de la cavité glénoïde Le système de coordonnées de référence est déterminé par son origine (O) et deux autres points de repères anatomiques de l'omoplate que sont le Trigonum Scapulae et l'angle inférieur. Il est par conséquent possible de définir à la fois une orientation relative de la glène par rapport à l'omoplate ainsi que l'inclinaison de la glène par rapport au système de coordonnées précité. A partir de ce repère, on peut donc positionner en trois dimensions la prothèse. Il est par conséquent possible de positionner l'axe de perçage à partir duquel va être indexé le gabarit de pose selon l'invention et de déterminer une orientation par un vecteur dans le repère défini précédemment.

On renvoie aux figures des dessins qui montrent les caractéristiques du gabarit de pose d'une prothèse d'épaule inversée ou non en vue d'assurer, comme indiqué précédemment, le perçage de la glène.

Ce gabarit désigné dans son ensemble par (1) comprend des parties d'appui aptes à coopérer avec les rebords de la glène (G). Les parties d'appui présentent, d'une manière opposée, des agencements de positionnement et de fixation temporaire coopérant avec les rebords postérieur (G1) et antérieur (G2) de la glène(G). Ces agencements (1b) et (1c) sont déterminés pour assurer une fixation temporaire de l'ensemble du gabarit par un effet de clipage et présentent des zones d'appuis délimitant deux sous-ensembles (1b1 - 1b2) et (1c1 - 1c2) séparant bord et rebord de la glène.

Ces agencements (1b) et (1c) sont constitués par des pattes orientées formant crochets et réalisées à partir d'une zone de liaison (1a) avec un manche (1d) La patte (1b), coopérant avec le bord antérieur de la glène, présente une zone d'appui (1b1) se prolongeant sur le rebord La patte antérieure (1b) présente facialement un profil sensiblement triangulaire. La patte opposée (1c), qui coopère avec le bord postérieur, est de longueur réduite en constituant un bourrelet profilé en section pour assurer l'effet de clipage.

Les parties d'appuis (1b) et (1c) coopèrent de manière unique avec les rebords de la glène, et sont prolongées par le manche de préhension (1d) agencé pour faire office de guide de perçage.

Le positionnement et l'orientation et du manche (1d) est déterminé en fonction du système de coordonnées tridimensionnelles résultant de la planification préopératoire, comme indiqué précédemment. Le manche de préhension (1d) est relié à la zone (1a) par une portée tronconique (1d1). La portée tronconique (1d1) et la partie inférieure du manche (1d), présentent deux ouvertures opposées (1e) et (1f), situées de part et d'autre des pattes (1b) et (1c). Ces ouvertures (1e) et (1f) permettent de visualiser le bon positionnement du gabarit et participent à améliorer l'élasticité pour obtenir l'effet de clipage recherché.

Comme indiqué, la partie supérieure du manche de préhension est agencée pour faire office de guide de perçage. Par exemple, ce guide de perçage est constitué par une zone pleine (1g) percée coxialement de manière concentrique à la zone (1a). A noter que la zone (1a) est constituée par un anneau de forme générale sensiblement circulaire correspondant à la partie inférieure de la cavité glénoïde.

On observe que les zones d'appui antérieure et postérieure sont construites en sélectionnant puis en extrudant de trois millimètres environ la surface sélectionnée de la structure 3D. De ces volumes 3D ont soustrait le volume augmenté de l'omoplate. Par volume augmenté, on considère un écart entre le modèle numérique 3D et le modèle réel qui est lié à la précision du scanner ou autre et à d'éventuels tissus biologiques résiduels non détectés par le scanner. Les essais effectués ont montré que cet écart était compris entre 0.3 et 1 millimètre environ.

A titre indicatif nullement limitatif, les caractéristiques dimensionnelles suivantes ont donné des résultats satisfaisants :
Longueur de gabarit considéré à partir de la partie d'appui (1a) jusqu'à l'extrémité du manche, environ 10cm permettant un positionnement depuis l'extérieur de l'incision.
▪ Longueur du gabarit de perçage (1g), environ 5cm
▪ Diamètre du manche, 15 millimètres environ ▪ Portée conique (1d1) faisant un angle d'environ 20° par rapport à l'axe du manche (1d)
▪ Epaisseur des pattes d'appui et de fixation (1b) (1c) de l'ordre de 3 à 4 millimètres environ. ▪ Forme triangulaire de la patte (1 b) faisant une base d'une longueur comprise entre 20 et 25 millimètres environ et une hauteur comprise entre 20 et 25 millimètres environ.
▪ Patte d'appui présentant une longueur d'environ 10 à 15 millimètres environ et une largeur de 5 à 10 millimètres environ.

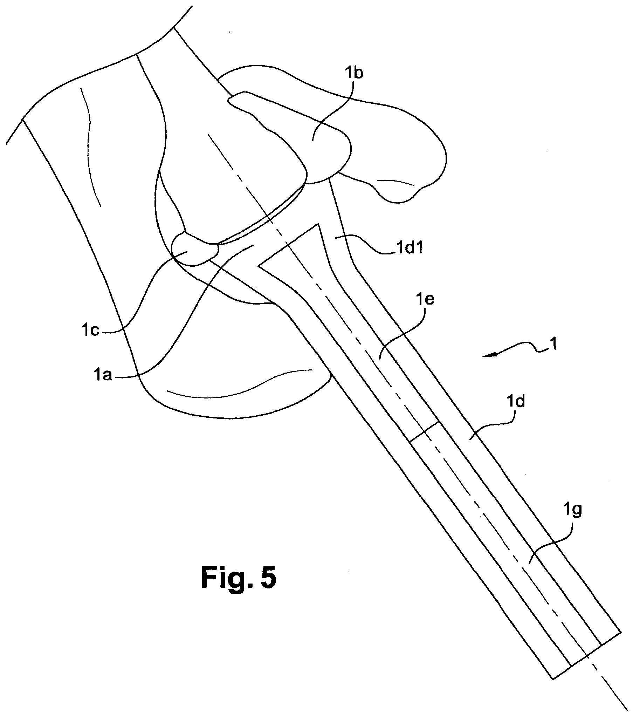

On renvoie à la figure 5 qui montre l'adaptation d'un gabarit de pose selon les caractéristiques de l'invention, par rapport à glène (G).

Les avantages ressortent bien de la description, en particulier on souligne et on rappelle que l'on obtient un gabarit de pose sur mesure qui reproduit pendant la chirurgie la planification peropératoire faite à partir d'un système de coordonnées tridimensionnelles lié à l'omoplate. Le système de coordonnées sera précisément retrouvé pendant la chirurgie par la coaptation de la forme anatomique osseuse avec la zone d'appui du gabarit de forme complémentaire.

## Revendications

1. Gabarit de pose (1) d'une prothèse d'épaule sur une glène (G), comprenant des parties d'appui aptes à coopérer avec la glène, ***caractérisé en ce que*** lesdites parties d'appui sont aptes à coopérer, de manière unique et sur mesure sur la structure anatomique osseuse du patient avec les rebords antérieur et postérieur de la glène, au moyen d'agencements de positionnement et de fixation temporaire délimitant des surfaces de contact (1b1 - 1b2) et (1c1 - 1c2) coopérant avec lesdits rebords postérieur et antérieur, les agencements étant constitués par des pattes orientées (1b) et (1c), chacune formant crochet, et réalisées à partir des surfaces de contact (1b1 - 1b2) et (1c1 - 1c2), la patte (1b) étant apte à coopérer avec le bord antérieur de la glène et la patte (1c) étant apte à coopérer avec le bord postérieur de ladite glène, lesdites pattes sont opposées et profilées pour assurer un effet de clippage et présentent des zones d'appui délimitant deux sous-ensembles (1b1-1b2) et (1c1-1c2) aptes à séparer bord et rebord de la glène, lesdites parties étant formées, à partir d'une zone de liaison (1a) avec un manche de préhension (1d) agencé pour faire office de guide de perçage (1g), le positionnement et l'orientation du manche (1d) sont déterminés par rapport à un système de coordonnées tridimensionnelles spécifique à l'omoplate du patient à opérer résultant d'une planification peropératoire qui se traduit par un axe de perçage défini par un vecteur et une origine, déterminant le centre (0) de la glène, et l'orientation dans l'espace du gabarit de pose sur mesure.

2. Gabarit de pose d'une prothèse d'épaule selon la revendication 1, ***caractérisé en ce que*** la zone de liaison (1a) avec le manche (1d) constitue un anneau de forme sensiblement circulaire.

3. Gabarit de pose d'une prothèse d'épaule selon l'une des revendications 1-*2, **caractérisé en ce que*** le manche de préhension (1d) est relié à la zone de liaison (1a) par une portée tronconique (1d1).

4. Gabarit de pose d'une prothèse d'épaule selon la revendication 3, ***caractérisé en ce que*** la portée tronconique (1d1) et la base du manche (1d) présentent deux ouvertures opposées (1e) et (1f) situées de part et d'autre des zones de contact constituées par les pattes (1b) et (1c), permettant une élasticité facilitant la mise en place dudit gabarit.

5. Gabarit de pose d'une prothèse d'épaule selon la revendication 1, ***caractérisé en ce que*** la patte (1c) coopérant avec le bord postérieur de la glène est de longueur réduite, apte à coopérer avec le rebord postérieur de la glène.

6. Gabarit de pose d'une prothèse d'épaule selon la revendication 1, ***caractérisé en ce que*** le guide de perçage (1g) est incorporé à la partie supérieure du manche.

7. Gabarit de pose d'une prothèse d'épaule selon l'une des revendications 1-*6, **caractérisé en ce que*** la longueur du manche est déterminée pour permettre le positionnement depuis l'extérieur de l'incision.

## Patentansprüche

1. Schablone (1) zum Einsetzen einer Schulterprothese in eine Gelenkpfanne (G) mit Auflageteilen, die in der Lage sind, mit der Gelenkpfanne zusammenzuwirken, ***dadurch gekennzeichnet,* dass** die Auflageteile in der Lage sind, einzigartig und maßgenau auf der anatomischen Knochenstruktur des Patienten mit der vorderen und hinteren Lippe der Gelenkpfanne mittels provisorischen Positionierung- und Befestigungsanordnungen, welchen mit den hinteren und vorderen Lippen zusammenwirkenden Kontaktflächen (1b1 - 1b2) und (1c1 - 1c2) begrenzen, und die Anordnungen von ausgerichteten Klauen (1b) und (1c) gebildet werden, die von den Kontaktflächen (1b1 - 1b2) und (1c1 - 1c2) aufgebaut sind, , wobei jede Klaue einen Haken bildet, und die Klaue (1b) in der Lage ist, mit dem vorderen Rand der Gelenkpfanne zusammenzuwirken und die Klaue (1c) in der Lage ist, mit dem hinteren Rand der Gelenkpfanne zusammenzuwirken, und die Klauen einander gegenüber liegen und zur Erzeugung einer Festklemmwirkung mit Profilen versehen sind, und Auflageteile aufweisen, welche zur fähige Trennung von Gelenkrand und Gelenklippe zwei Untereinheiten (1b1-1b2) und (1c1-1c2) abgrenzen, wobei die Teile durch einen Verbindungsbereich (1a) mit einem als Bohrführung (1g) fungierenden Greifschaft (1d) geprägt sind, die Positionierung und die Ausrichtung des Schafts (1d) werden im Rahmen einer intraoperativen Planung über ein dreidimensionales Koordinatensystem bestimmt, das spezifisch auf das Schulterblatt des zu operierenden Patienten ausgelegt ist, was zu einer durch einen Vektor und einen Ursprung definierten Bohrachse führt, die die Mitte (0) der Gelenkpfanne und die räumliche Ausrichtung der maßgefertigten Einsetzschablone festgelegt.

2. Schablone zum Einsetzen einer Schulterprothese nach Anspruch 1, ***dadurch gekennzeichnet,* dass** der Verbindungsbereich (1a) zum Schaft (1d) einen im Wesentlichen kreisförmigen Ring darstellt.

3. Schablone zum Einsetzen einer Schulterprothese nach einem der Ansprüche 1-2, ***dadurch gekennzeichnet,* dass** der Greifschaft (1d) mit dem Verbindungsbereich (1a) über eine kegelstupmpfförmige Auflagefläche (1d1) verbunden ist.

4. Schablone zum Einsetzen einer Schulterprothese nach Anspruch 3, ***dadurch gekennzeichnet,* dass** die kegelstumpfförmige Auflagefläche (1dl) und der Schaftstumpf (1d) zwei einander gegenüberliegende Öffnungen (1e) und (1f) aufweisen, die beiderseits der durch die Klauen (1b) und (1c) gebildeten Kontaktbereiche angeordnet sind, wodurch eine Elastizität erzielt wird, die das Einsetzen der Schablone erleichtert.

5. Schablone zum Einsetzen einer Schulterprothese nach Anspruch 1, ***dadurch gekennzeichnet,* dass** die mit dem hinteren Rand der Gelenkpfanne zusammenwirkende Klaue (1c) eine geringere Länge aufweist, um mit der hinteren Lippe der Gelenkpfanne zusammenwirken zu können.

6. Schablone zum Einsetzen einer Schulterprothese nach Anspruch 1, ***dadurch gekennzeichnet,* dass** die Bohrführung (1g) im oberen Bereich des Schafts integriert ist.

7. Schablone zum Einsetzen einer Schulterprothese nach Anspruch 1, ***dadurch gekennzeichnet,* dass** die Schaftlänge festgelegt ist, um die Positionierung von außerhalb des Einschnitts zu ermöglichen.

## Claims

1. Installation jig (1) of a shoulder prosthesis on a glenoid (G), comprising bearing portions adapted to cooperate with the glenoid, **characterized in that** said bearing parts are adapted to cooperate, in a single and customized way, on the patient's bone anatomical structure with the anterior and posterior rims of the glenoid, by means of temporary positioning and fixing arrangements bounding contact surfaces (1b1 - 1b2) and (1c1 - 1c2) cooperating with said posterior and anterior rims, the arrangements being comprised of oriented lugs (1b) and (1c), each one forming hook, and made from the contact surfaces (1b1 - 1b2) and (1c1 - 1c2), the lug (1b) being able to cooperate with the anterior edge of the glenoid, and the lug (1c) being able to cooperate with the rear edge of said glenoid, said lugs are opposed and contoured to ensure a clipping effect and have bearing areas defining two subsets (1b1-1b2) and (1c1-1c2) able to separate edge and rim of the glenoid, said portions being formed, from a connecting region (1a) with a gripping handle (1d) arranged to act as drill guide (1g), the positioning and orientation of the handle (1d) are determined with respect to a three-dimensional coordinate system specific to the patient's scapula to operate, as a result of an preoperative planning which results in a drilling axis defined by a vector and an origin, determining the center (0) of the glenoid, and the orientation in space of the customized installation jig.

2. Installation jig of a shoulder prosthesis according to claim 1, **characterized in that** the connecting area (1a) with the handle (1d) is a substantially circular ring.

3. Installation jig of a shoulder prosthesis according to anyone of claims 1-2, **characterized in that** the gripping handle (1d) is connected to the connecting area (1a) by a frustoconical bearing surface (1d1).

4. Installation jig of a shoulder prosthesis according to claim 3, **characterized in that** the frustoconical bearing surface (1d1) and the base of the handle (1d) have two opposed openings (1e) and (1f) located on both sides of the contact areas formed by the lugs (1b) and (1c), allowing elasticity facilitating the installation of said jig.

5. Installation jig of a shoulder prosthesis according to claim 1, **characterized in that** the lug (1c) cooperating with the rear edge of the glenoid is of reduced length, able to cooperate with the rear edge of the glenoid.

6. Installation jig of a shoulder prosthesis according to claim 1, **characterized in that** the drill guide (1g) is incorporated to the upper portion of the handle.

7. Installation jig of a shoulder prosthesis according to anyone of claims 1-6, **characterized in that** the handle length is determined to allow positioning from outside the incision.
